(19) Europäisches Patentamt
European Patent Office
Office européen des brevets

(11) Veröffentlichungsnummer: **0 076 493**
**B1**

# (12) EUROPÄISCHE PATENTSCHRIFT

(45) Veröffentlichungstag der Patentschrift: **06.08.86**

(21) Anmeldenummer: **82109101.4**

(22) Anmeldetag: **01.10.82**

(51) Int. Cl.[4]: **C 11 B 9/00** // C07C47/228, C07C29/14, C07C33/20, C07C69/157, C07C121/66

## (54) Verwendung von 1,1-Di(C1-C6-alkyl)-2-phenyl-ethan-Derivaten als Riechstoffe.

(30) Priorität: **02.10.81 DE 3139358**

(43) Veröffentlichungstag der Anmeldung: **13.04.83 Patentblatt 83/15**

(45) Bekanntmachung des Hinweises auf die Patenterteilung: **06.08.86 Patentblatt 86/32**

(84) Benannte Vertragsstaaten: **CH FR GB LI NL**

(56) Entgegenhaltungen:
**EP - A - 0 032 576**
**GB - A - 1 140 014**
**GB - A - 1 569 424**

**S. ARCTANDER: "Perfume and flavor chemicals", CAroma Chemicals) I, 1969, Montclair, N.J., US;**

(73) Patentinhaber: **Dragoco Gerberding & Co. GmbH, Dragocostrasse 1, D-3450 Holzminden (DE)**

(72) Erfinder: **Brunke, Ernst-Joachim, Dr., Holbeinstrasse 6, D-3450 Holzminden (DE)**
Erfinder: **Klein, Erich, Dr., Wiesenweg 50, D-3450 Holzminden (DE)**

(74) Vertreter: **Deufel, Paul, Dr. et al, Patentanwälte Müller-Boré, Deufel, Schön, Hertel Lewald, Otto Isartorplatz 6 Postfach 26 02 47, D-8000 München 26 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Gegenstand der Erfindung ist die Verwendung der 1,1-Dialkyl-2-phenyl-ethan-Derivate Aa–e, worin R eine Formyl-, Hydroxymethyl-, Acetoxymethyl-, Nitril- oder Formoxymethylgruppe bedeutet, als Riechstoff oder Riechstoffbestandteil von Riechstoff-Mischungen für kosmetische und technische Parfümierungen.

Die Erfindung wird zunächst anhand der α,α-Dimethyl-Derivate 1a–1d erläutert.

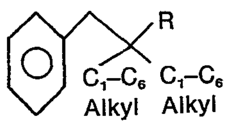

A

a: R = CHO
b: R = $CH_2OH$
c: R = $CH_2OAc$
d: R = CN
e: R = $CH_2OCHO$

Für die Parfümerie sind Riechstoffe mit blumiger Geruchscharakteristik von grossem Interesse. Da die Destillate oder Extrakte aus Pflanzenteilen mit dem jeweils charakteristischen Duft entweder sehr kostspielig sind – wie z.B. bei Rosen-Öl oder Jasmin-Absolue-, oder aufgrund ihrer Instabilität nicht zu gewinnen sind – wie bei Flieder oder Maiglöckchen –, ist man seit Bestehen der Riechstoff-Industrie bestrebt, synthetische Ersatzprodukte darzustellen. Während es für die meisten der verschiedenen geruchsprägenden Substanzen aus den stabilen Pflanzen-Destillaten oder -Extrakten bereits chemische Partial- oder Totalsynthesen gibt, die auch oft technisch realisierbar sind, kann bei den instabilen Naturdüften wie Maiglöckchen oder Flieder nur auf technisch stabile, synthetische Ersatzprodukte zurückgegriffen werden, die zwar eine vom Naturstoff abweichende Struktur, aber einen ähnlichen Duft aufweisen sollten. Derartige vielfach verwendete synthetische Riechstoffe mit blumigem Charakter sind z.B. Dimethylbenzyl-carbinol (2) oder Phenylethyl-dimethyl-carbinol (3). Der Duft der Verbindung 2 wird beschrieben als warm krautig-blumig, leicht animalisch mit einer Nebennote nach frisch geschnittenem Holz und Anklängen von Lilien- und Holunderblütenduft (S. Arctander, Perfume and Flovor Chemicals, 1969, Nr. 989). Die Verbindung 3 (Arctander, Nr. 1043) besitzt einen blumig grünen, schwach krautigen und öligen Duft, der an Lilie und Hyazinthe erinnert. Beide Verbindungen 2, 3 sind nur durch Grignard-Reaktionen zugänglich, die einen relativ hohen technischen Aufwand erfordern.

Die zu den tertiären Alkoholen 2 und 3 analogen sekundären Alkohole werden in der Parfümerie nicht verwendet, wohl aber die entsprechenden primären Alkohole 4 und 5. Allerdings besitzen diese Verbindungen erheblich von 2, 3 abweichende Geruchsnoten.

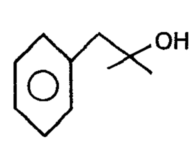

2

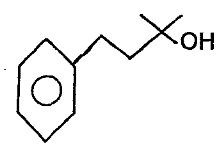

3

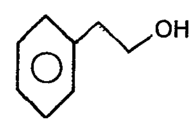

4

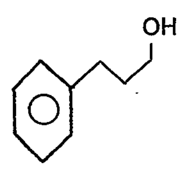

5

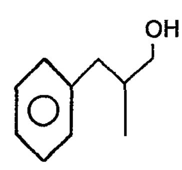

6

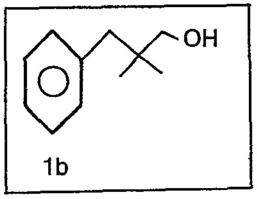

1b

So wird der Duft des in grossen Mengen verwendeten Phenylethylalkohols (4) als «mild und warm, Rosen- und Honig-artig» beschrieben (Arctander, Nr. 2513). Der Dihydrozimtalkohol (5) riecht etwas zimtartig, bzw. «warm und mild, balsamisch-blumig, süss» (Arctander, Nr. 2589). Der durch Aldolkondensation von Benzaldehyd und Propionaldehyd sowie anschliessende Hydrierung dargestellte α-Methyl-dihydro-zimtalkohol (6) besitzt einen sehr schwachen, zimtartigsüssen, leicht holzigen Geruch und wird nicht als Riechstoff verwendet.

Es ist daher überraschend, dass der α,α-dimethyl-substituierte primäre Alkohol 1b einen stark ausstrahlenden und angenehm blumigen Duft in Richtung Maiglöckchen und Hyazinthe aufweist, der an bestimmte Geruchsaspekte der tertiären Carbinole erinnert, diese jedoch in Parfümkompositionen an natürlicher Ausstrahlung übertrifft. Die α,α-Dimethyl-Verbindung 1b ist in der chemischen Literatur beschrieben worden, ohne dass ihre besonderen olfaktorischen Eigenschaften erkannt oder erwähnt worden sind. [V.G. Purohit und R. Subramanian, Chem. and Ind. 1978, 731; P. Warrick und W.H. Saunders, J. Am. Chem. Soc., 84, 4095 (1962). A. Haller und E. Bauer, Ann. Chim., 9, 15 (1918)]. Es wurde gefunden, dass sich die Verbindung 1b aufgrund ihrer Geruchseigenschaften sehr gut in Parfümöle blumigen Typs einarbeiten lässt und diesen mehr natürliche Ausstrahlung verleiht.

Der Aldehyd 1a besitzt einen starken aldehydisch-grünen Geruch der vor allem in geringen Dosierungen vorteilhaft in Parfümöle eingearbeitet werden kann. Die Darstellung des α, α-dimethyl-substituierten Aldehyds 1a wurde von H.K. Dietl und K.C. Brannock (Tetrahedron Letters 1973, 1273) beschrieben, ohne dass auf die Geruchseigenschaften der Verbindung eingegangen worden ist.

Durch übliche Veresterung wurden aus dem primären Alkohol 1b verschiedene Ester dargestellt, von denen das Acetat 1c und das Formiat 1e besondere olfaktorische Effekte aufweisen. Die Geruchsnoten von 1c können als «holzig, süsskrautig, blumig» und die von 1e als «holzig, krautig, grün» beschrieben werden. Das Nitril 1d besitzt einen krautig-balsamischen Duft mit blumigen und fruchtigen Nebennoten. Die Verbindungen 1a–1d sind aus Grundchemikalien auf relativ einfachem Weg zugänglich und können daher in beliebiger Menge produziert werden.

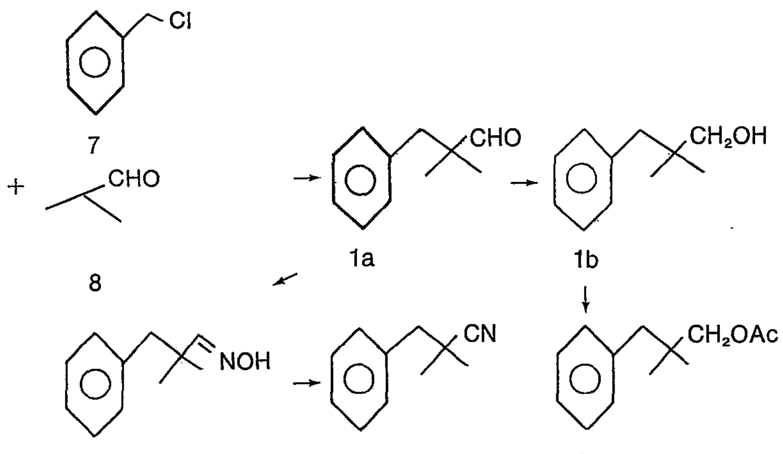

7 8 1a 1b 9 1d 1c

Die Darstellung der Substanzen 1a–d erfolgte ausgehend von Benzylchlorid (7), das in einer «phase-transfer»-Reaktion mit Isobutyraldehyd (8) in an sich bekannter Weise zu dem Aldehyd 1a umgesetzt wurde. Diese Reaktion ist unter Verwendung von 50%ig. wässriger Natronlauge und katalytischen Mengen an Tetrabutylammoniumjodid beschrieben worden (H.K. Dietl und K.C. Brannock, Tetrahedron Letters 1973, 1273). Es wurde gefunden, dass anstelle des von Dietl und Brannock eingesetzten, kostspieligen Tetrabutylammonium-jodids auch das preiswerte Trioctylmethyl-ammonium-chlorid (Aliquat 336) verwendet werden kann. Die Reaktion wird bei erhöhter Temperatur, vorzugsweise bei Siedetemperatur, in einem Zweiphasen-System, bestehend aus Toluol und 50%iger NaOH durchgeführt. Durch übliches Aufarbeiten und Destillation wurde der Aldehyd 1a in ca. 60% Ausbeute erhalten. Die Reduktion des Aldehyds 1a zu dem primären Alkohol 1b erfolgte vorzugsweise durch katalytische Hydrierung unter Verwendung von Raney-Nickel oder Kupfer-chromit in unverdünntem Zustand oder unter Verwendung von polaren aprotischen Lösungsmitteln wie Methanol. Weiterhin wurde die Reduktion unter Verwendung von Hydriden wie Natriumborhydrid oder Lithiumaluminiumhydrid unter Einhaltung üblicher Reaktionsbedingungen ausgeführt.

Die Veresterung erfolgte in an sich bekannter Weise durch Umsetzen des Alkohols 1b mit Säureanhydriden in Gegenwart säurebindender Substanzen wie beispielsweise Natriumcarbonat oder Kaliumcarbonat. Durch Umsetzung von 1b mit Acetanhydrid/Natriumcarbonat wurde das Acetat 1c dargestellt.

Das Nitril 1d wurde in an sich bekannter Weise durch Einwirkung von Hydroxylamin auf 1a in einem basischen Medium unter Bildung des Oxims 9 und dessen nachfolgende Dehydratisierung durch Einwirkung von Acetanhydrid in der Siedehitze dargestellt.

Die für die Darstellung der Verbindungen 1a–1d beschriebenen Reaktionsschritte lassen sich auf homologe Verbindungen übertragen. So lassen sich α-alkyl-substituierte Aldehyde mit maximal 10 Kohlenstoff-Atomen wie z.B. 2-Methyl-butanal, 2-Methylpentanal, 2-Ethyl-butanal, 2-Ethyl-pentanal, 2-Ethyl-hexanal, 2-Propylpentanal vorteilhaft unter Verwendung von «phase-transfer»-Katalysatoren, vorzugsweise Trioctyl-methyl-ammoniumchlorid und Alkalihydroxiden, mit Benzylchlorid alkylieren. Die hierbei erhaltenen Aldehyde der allgemeinen Formel Aa besitzen frische und z.T. holzig-krautige Noten. Die in Analogie zu 1c dargestellten Acetate der allgemeinen Formel Ac besitzen holzige und süss-krautige Geruchsnoten, während die in Analogie zu 1b erhaltenen primären Alkohole der allgemeinen Formel Ab mildblumige, z.T. frische Geruchsaspekte aufweisen. Die in Analogie zu 1d erhaltenen Nitrile der allgemeinen Formel Ad besitzen süss-blumige und krautige und die Formiate Ae frisch-holzige Noten.

Das Verfahren zur Herstellung der erfindungsgemäss verwendeten Verbindungen kann ganz allgemein wie folgt zusammengefasst werden: Man setzt Benzylchlorid mit einem α-verzweigten aliphatischen Aldehyd mit bis zu 10 Kohlenstoffatomen in einer an sich bekannten «phase-transfer»-Reaktion unter Verwendung von Alkalihydroxiden und quaternären Ammoniumsalzen, vorzugsweise Trioctyl-methyl-Ammoniumchlorid zum Aldehyd Aa um;

Der hierbei erhaltene Aldehyd Aa wird gegebenenfalls in an sich bekannter Weise durch katalyti-

sche Reduktion unter Verwendung von Übergangsmetall-Katalysatoren, vorzugsweise Raney-Nickel oder Kupferchromat zum Alkohol Ab reduziert;

Der hierbei erhaltene Alkohol Ab wird gegebenenfalls in an sich bekannter Weise zu Ac verestert, wobei man Acetanhydrid in Gegenwart säurebindender Mittel wie Natrium- oder Kaliumcarbonat oder -acetat einwirken lässt, oder

Der in Stufe 1 erhaltene Aldehyd Aa wird gegebenenfalls in an sich bekannter Weise mit Hydroxylamin zu dem entsprechenden Oxim umgesetzt und dieses vorzugsweise durch Einwirkung von Acetanhydrid in der Siedehitze zum Nitril Ad dehydratisiert;

Der in Stufe 2 erhaltene Alkohol Ab wird gegebenenfalls in an sich bekannter Weise zu Ae umgesetzt.

Mit zunehmendem Molekulargewicht werden die Geruchsaspekte der Verbindungen Aa–d schwerer und weniger hell, z.T. auch in der Intensität etwas schwächer als die der Dimethyl-substituierten Verbindungen 1a–d. Allerdings treten die fixierenden und abrundenden Geruchseffekte in Parfumkompositionen bei höherem Molekulargewicht stärker hervor. Aufgrund ihrer Geruchscharakteristiken, ihrer fixierenden Eigenschaften und ihrer Stabilität in kosmetischen und technischen Medien können die Verbindungen der allgemeinen Formeln Aa–Ae vorteilhaft zum Parfümieren kosmetischer und technischer Produkte verwendet werden.

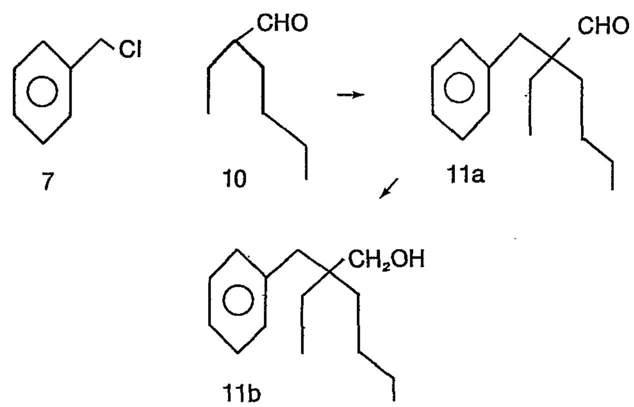

7 10 11a

11b

Der der allgemeinen Formel Aa entsprechende Aldehyd 11a mit 15 Kohlenstoffatomen wurde aus Benzylchlorid (7) und α-Ethylhexanal (10) nach der für 1a gegebenen Vorschrift dargestellt. Die Geruchsnoten können mit hell, krautig, fruchtig (Agrumenaspekte) beschrieben werden. Durch Reduktion wurde analog zu 1b der Alkohol 11b mit mild-blumiger Note erhalten.

Die anschliessenden Beispiele erläutern die Darstellung der Verbindungen 1a–d und deren Anwendung in Parfümölen.

Beispiel 1
Darstellung von
2,2-Dimethyl-3-phenyl-propanal-1 (1a):

Unter Rühren wurde eine Lösung von 224 g (4 mol) KOH und 10 g Trioctyl-methyl-ammoniumchlorid (Aliquat 336) in 125 g Wasser mit 200 ml Toluol versetzt und zum Sieden erhitzt. Bei fortwährendem Sieden wurde eine Lösung von 402 g (3.2 mol) Benzylchlorid (7) und 256 g (3.6 mol) Isobutyraldehyd innerhalb von 1 Stde. zugetropft. Nach 7 Stdn. Rühren bei Siedetemp. und Abkühlen auf Raumtemp. wurde mit 300 g Wasser ausgedünnt. Die wässrige Phase wurde mit Toluol extrahiert. Die vereinigten organischen Phasen wurden neutralgewaschen und eingeengt. Das Rohprodukt (510 g gelbes Öl) ergab bei Destillation über eine 60 cm Glasfüllkörperkolonne 354 g (69%) 1a als farbloses Öl; Kp. (2 mm) = 73 °C – NMR ($CCl_4$) : δ = 0.92, s, 6H (2,2–$CH_3$), 2.68, s ($CH_2$–3), 7.0–7.4, m (Phenyl–H), 9.65 ppm, s (CH–1). – IR: 1730, 2740 $cm^{-1}$ (Aldehyd). – MS: m/e (%) = 162 (20, $M^+$), 147 (4), 133 (7), 115 (5), 105 (4), 91 (100). $C_{11}H_{14}O$ (162, 2).

Beispiel 2
Darstellung von
2,2-Dimethyl-3-phenyl-propanol (1b):

In einem Autoklav wurden 354 g 2,2-Dimathyl-3-phenyl-propanal (1a) mit 2 g Kupferchromit (G–79) bei 150 °C und 120 bar bis zur Aufnahme von 1 Äquivalent Wasserstoff hydriert. Nach Entspannen des Autoklavs wurde der Katalysator abgetrennt. Destillation über eine 60 cm Füllkörperkolonne ergab 320 g (90%) 2,2-Dimethyl-3-phenyl-propanol (1b) als farbloses, erstarrendes Öl. Kp. (2 mm) = 90 °C, Schmp. 34–36 °C. – NMR ($CCl_4$) : δ = 0.88, s, 6H (2,2–$CH_3$), 2.55, s ($CH_2$–3), 3.37, s ($CH_2$–1), 7.1–7.3, br. s (Phenyl–H). – IR: 3400 $cm^{-1}$ (O–H). – MS: m/e (%) = 164 (16, $M^+$), 133 (5), 131 (4), 117 (6), 115 (8), 105 (4), 92 (100), 91 (83). $C_{11}H_{16}O$ (164.2).

Beispiel 3
Darstellung von
2,2-Dimethyl-3-phenyl-propyl-1-acetat (1c):

Eine Lösung von 100; 2,2-Dimethyl-3-phenyl-propan-1-ol (1b) und 20 g Natriumacetat in 100 g Essigsäureanhydrid wurde 3 Stdn. zum Sieden erhitzt. Nach üblicher Aufarbeitung wurde das Rohprodukt (105 g hellgelbes Öl) über eine 30 cm Glasfüllkörperkolonne destilliert. Man erhielt 90 g 1c als farbloses Öl; Kp. (2 mm) = 90 °C. – NMR ($CCl_4$) : δ = 0.87, s, 6H (2,2–$CH_3$), 1.98, s (–O–CO–$\underline{CH_3}$), 2.53, s ($CH_2$–3), 3.73, s ($CH_2$–1),

6.9–7.2 ppm, m (Phenyl–H). – IR: 1735 $cm^{-1}$ (Acetat). – MS: m/e (%): 206 ($M^+$), 146 (27), 131 (22), 115 (16), 91 (35), 43 (100).
$C_{13}H_{18}O_2$ (206.3).

Beispiel 4
Darstellung von
2,2-Dimethyl-3-phenyl-propionitril (1d):

Bei 20–30 °C wurden einer Lösung von 22.7 g (0.25 mol) Hydroxylaminhydrogensulfat in 40 ml Methanol und 20 ml Wasser unter Rühren 36 g wässrige Natronlauge (33%) zugegeben. Anschliessend liess man 40.5 g (0.25 mol) 2,2-Dimethyl-3-phenyl-propanal (1a) zulaufen und rührte die Reaktionsmischung 3 Stdn. bei Raumtemperatur. Zur Aufarbeitung wurde mit 150 ml Wasser ausgedünnt und mit Methylenchlorid extrahiert. Nach Einengen verblieben ca. 45 g Oxim 9, die innerhalb von 20 min zu 80 g siedenden Acetanhydrids hinzugegeben wurden. Nach 5 Stdn. Rühren bei Siedetemp. wurde mit 200 ml Wasser ausgedünnt, mit Benzin extrahiert und eingeengt. Destillation des Rohprodukts (6.40 g) über eine kurze Kolonne und anschliessende Kristallisation aus Ethanol ergab 20 g 1d vom Schmp. 55–56 °C. – NMR ($CCl_4$) : δ = 1.27, s, 6H (2,2–$CH_3$), 2.71, s ($CH_2$–3), 7.1–7.3 ppm (Phenyl–H). – IR: 2230 $cm^{-1}$ (Nitril). – MS: m/e (%) = 159 (11, $M^+$), 146 (3), 132 (3), 117 (6), 115 (3), 91 (100), 65 (9).
$C_{11}H_{13}N$ (159.2).

Beispiel 5

| Parfümöl mit Maiglöckchen-Duft | a | b |
|---|---|---|
| 1-Formyl-4-(4-methyl-4-hydroxy-amyl)-cyclohexen-3 [Lyral®] | 190 | 190 |
| α-Hexylzimtaldehyd | 170 | 170 |
| Citronellol | 145 | 145 |
| Phenylethyl-dimethyl-carbinol (3) | 130 | – |
| 2,2-Dimethyl-3-phenyl-propanol (1b) | – | 130 |
| 2-Phenyl-ethanol | 120 | 120 |
| Lilial® (p. tert. Butyl-α-methylhydrozimtaldehyd) | 40 | 40 |
| Indol, 10% in DPG | 35 | 35 |
| Cyclopentadecanolid, 1% in DPG | 25 | 25 |
| 1-(3-Hydroxy-hexyl)-2,2,6-trimehyl-cyclohexan | 15 | 15 |
| cis-3-Hexenol-1, 10% in DPG | 10 | 10 |
| cis-3-Hexenyl-1-acetat, 10% in DPG | 10 | 10 |
| Jasmin-Absolue, 10% in DPG | 10 | 10 |
| | 900 | 900 |

Das Parfümöl der Mischung a besitzt einen ausgewogenen Maiglöckchen-Duft. Die Mischung b, in der anstelle des Carbinols 3 der erfindungsgemässe Alkohol 1b eingesetzt wurde, besitzt ebenfalls einen harmonischen Maiglöckchen-Duftkomplex, der sich jedoch gegenüber der Mischung a durch mehr Fülle und natürliche Ausstrahlung auszeichnet.

Beispiel 6

| Parfümöl mit Fougère-Note | a | b |
|---|---|---|
| Geraniol | 100 | 100 |
| Citronellol | 100 | 100 |
| Benzylacetat | 90 | 90 |
| Phenylethylalkohol | 80 | 80 |
| Patchouli-Öl, Singapore | 80 | 80 |
| α-Pentyl-zimtaldehyd | 75 | 75 |
| α-Methyl-jonon | 45 | 45 |
| Pentyl-salicylat | 45 | 45 |
| Lavandinöl, Abrialis | 45 | 45 |
| Bergamotte-Öl, Reggio | 45 | 45 |
| Terpineol | 40 | 40 |
| Coumarin | 30 | 30 |

| | | |
|---|---|---|
| 1-(4-Hydroxy-3-methyl-butyl)-2,2,3-trimethyl-cyclopenten-2 (Brahmanol®) | 30 | 30 |
| 1,3,4,6,7,8-Hexyhydro-4,6,6,7,8,8-hexamethyl-cyclopenta-γ-2-benzopyran (Galaxolide®) | 25 | 25 |
| Benzoe-Resinoid, Siam | 10 | 10 |
| Styrax-Extrakt | 10 | 10 |
| Eichenmoos-Extrakt | 10 | 10 |
| Dipropylenglykol | 130 | – |
| 2,2-Dimethyl-3-phenyl-propyl-1-acetat (1c) | – | 130 |
| | 990 | 990 |

Das Parfümöl der Mischung a hat einen Duftkomplex vom Fougère-Typ mit holzigen, herbsüssen Aspekten. Das Parfümöl b, das die neue Verbindung 1c enthält, besitzt einen Duftkomplex ähnlichen Typs, in dem jedoch eine süss-pudrige Note betont wird und der zugleich deutlich abgerundet und harmonischer wirkt.

Beispiel 7

| Parfümöl "Flieder" | a | b | c |
|---|---|---|---|
| Terpineol | 350 | 350 | 350 |
| 2,2-Dimethyl-3-phenyl-propanol (1b) | – | 150 | – |
| Dipropylenglykol | 150 | – | 100 |
| Phenylethylalkohol | 100 | 100 | 100 |
| 2,2-Dimethyl-3-phenyl-propionitril (1d) | – | – | 50 |
| Benzylacetat | 50 | 50 | 50 |
| Lilial | 50 | 50 | 50 |
| Geraniol | 45 | 45 | 45 |
| Methyl-dihydrojasmonat | 40 | 40 | 40 |
| Linalool | 40 | 40 | 40 |
| Zimtalkohol | 20 | 20 | 20 |
| p-Tolylacetaldehyd, 50% in DPG | 35 | 35 | 35 |
| Farnesol | 20 | 20 | 20 |
| Benzylisoeugenol | 20 | 20 | 20 |
| Citronellol | 10 | 10 | 10 |
| Linalooloxid | 10 | 10 | 10 |
| Indol, 10% in DPG | 5 | 5 | 5 |
| cis-3-Hexenol, 10% in DPG | 5 | 5 | 5 |
| Isojasmon | 3 | 3 | 3 |
| Isoeugenol | 2 | 2 | 2 |
| cis-3-Hexenylacetat, 10% in DPG | 2 | 2 | 2 |
| Eugenolmethylether | 2 | 2 | 2 |
| p-Kresol, 10% in DPG | 1 | 1 | 1 |
| | 960 | 960 | 960 |

Das Parfümöl a besitzt einen ausgewogenen Flieder-Duft. Das durch Austausch des geruchlosen Dipropylenglykol und Einsetzen von 15% des Carbinols 1b erhaltene Parfümöl hat einen noch stärker ausstrahlenden und natürlich wirkenden Fliederduft. Die Einarbeitung von 5% des Nitrils 1d führt unter Betonung der krautig-grünen Aspekte zu einem originellen blumig-frischen Duftkomplex.

Beispiel 8

| Parfümöl mit pudrig-süssem Charakter | | | |
|---|---|---|---|
| | a | b | c |
| Bergamottöl | 350 | 350 | 350 |
| Amylsalicylat | 130 | 130 | 130 |
| Benzylacetat | 100 | 100 | 100 |
| Anisaldehyd | 50 | 50 | 50 |
| Heliotropin | 50 | 50 | 50 |
| Benzoe-Extrakt Siam | 30 | 30 | 30 |
| Geraniumöl Bourbon | 30 | 30 | 30 |
| Moschus, Ambrette | 30 | 30 | 30 |
| Phenylethylalkohol | 30 | 30 | 30 |
| Coumarin | 20 | 20 | 20 |
| Nerol | 10 | 10 | 10 |
| Citronellol | 10 | 10 | 10 |
| Vanillin | 5 | 5 | 5 |
| Methylchavicol | 5 | 5 | 5 |
| Eichenmoos-Extrakt, 50% in DPG | 4 | 4 | 4 |
| Citral | 2 | 2 | 2 |
| Isojasmon | 2 | 2 | 2 |
| Thymol, 10% in DPG | 1 | 1 | 1 |
| 3-Methyl-5-propyl-cyclohexenon | 1 | 1 | 1 |
| Dipropylenglykol | 40 | – | – |
| 2,2-Dimethyl-3-phenyl-propionitril (1d) | – | 40 | – |
| 2,2-Dimethyl-3-phenyl-propanal (1a) | – | – | 40 |
| | 900 | 900 | 900 |

Das Parfümöl der Zusammensetzung a besitzt eine klassische süss-pudrige Note mit holzigen und würzigen Aspekten. Die durch Zugabe des erfindungsgemässen Nitrils 1d erhaltene Mischung b wirkt harmonischer und weicher unter gleichzeitiger Betonung süss-würziger Nebennoten. Durch Zugabe von 5% des erfindungemässen Aldehyds 1a (Mischung c) wurde ein neuer Duftakkord erhalten, der sich durch frische Ausstrahlung bei holzig-süssem Grundgeruch und grün-krautigen Nebennoten auszeichnet.

Beispiel 9
Darstellung von
2-Ethyl-2-benzyl-hexanal-1 (11a):

In Analogie zu der in Beispiel 1 gegebenen Vorschrift wurde aus Benzylchlorid (7) und α-Ethylhexanal (10) der Aldehyd 11a in einer Ausbeute von 62% als farbloses Öl gewonnen; Kp. (2 mm) = 110 °C. – NMR ($CCl_4$) : $\delta$ = 0.84, t, 6H ($-CH_2-\underline{CH_3}$), 2.75, s ($\Phi -\underline{CH_2}-$), 7.0–7.2, m (aromat. H), 9.7 ppm (–CHO). – IR: 2750, 1725 $cm^{-1}$ (Aldehyd). –MS: m/e (%) = 218 (2, $M^+$), 189 (6), 162 (7), 161 (7), 133 (4), 119 (5), 105 (7), 92 (18), 91 (100).
$C_{15}H_{22}O$ (218.3).

Beispiel 10
Darstellung von
2-Ethyl-2-benzyl-hexanol-1 (11b):

In Analogie zu der in Beispiel 2 gegebenen Vorschrift wurde aus 11a der Alkohol 11b in einer Ausbeute von 90% als farbloses Öl mit Kp. (2 mm) = 125 °C erhalten. NMR ($CCl_4$) : $\delta$ = 0.88, br. t, CH ($-CH_2-\underline{CH_3}$), 2.58, s ($\Phi -\underline{CH_2}-$), 3.25, s ($-\underline{CH_2}-OH$), 7.13 ppm, s (aromat. H). – IR: 3400 $cm^{-1}$ (OH). – MS: m/e (%) = 220 (4, $M^+$), 128 (12), 92 (100), 91 (62), 69 (47).
$C_{15}H_{24}O$ (220.3).

Beispiel 11
Parfümöl mit frischer Agrumen-Note

| | |
|---|---|
| Citronenöl | 250 |
| Orangenöl, Florida | 250 |
| Bergamottöl | 230 |
| Lavendelöl | 50 |
| Neroliöl | 50 |
| Rosmarinöl, tunesisch | 30 |
| 2-Ethyl-1-ethylendioxy-hexan | 20 |
| Geraniol | 10 |
| α-Hexyl-zimtaldehyd | 5 |
| Moschus, Keton | 5 |
| | 900 |

Dieses Parfümöl besitzt einen harmonischen Geruchskomplex vom Agrumen-Typ mit frischen grün-krautigen Noten. Nach Zugabe von 100 Teilen 2-Benzyl-2-ethyl-hexanal (11a) wirkt das Parfümöl feiner und natürlicher; gleichzeitig erfährt der Duftkomplex eine deutliche Fixierung.

**Patentanspruch**

Verwendung von 1,1-Di($C_1$–$C_6$-alkyl)-2-phenyl-ethan-Derivaten der Formel A, wobei R eine Formyl-, Hydroxymethyl-, Formoxymethyl-, Acetoxymethyl oder Nitrilgruppe bedeutet und die Gesamtzahl der Kohlenstoffatome in den beiden Alkylsubstituenten zusammen maximal 8 beträgt, als Riechstoffe oder Riechstoffbestandteil von Parfüm-Kompositionen für kosmetische oder technische Parfümierungen.

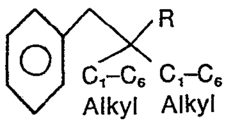

A

a: R = CHO
b: R = $CH_2OH$
c: R = $CH_2OAc$
d: R = CN
e: R = $CH_2OCHO$

**Claim**

The use of 1,1-Di($C_1$–$C_6$-alkyl)-2-phenyl-ethane derivatives of formula A

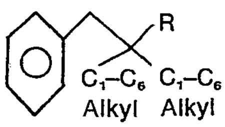

A

a: R = CHO
b: R = $CH_2OH$
c: R = $CH_2OAc$
d: R = CN
e: R = $CH_2OCHO$

wherein R is a formyl-, hydroxymethyl-, formoxymethyl-, acetoxymethyl- or nitrilo group and the total number of carbon atoms in both alkyl substituents together is 8 at most, as aroma chemicals or flavoring ingredient of perfume compositions for cosmetic or technical flavorings.

**Revendication**

Utilisation de dérivés de 1,1-di(alkyle en $C_1$–$C_6$)-2-phényl-éthane de formule A,

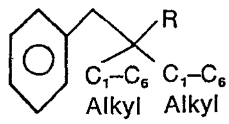

A

a: R = CHO
b: R = $CH_2OH$
c: R = $CH_2OAc$
d: R = CN
e: R = $CH_2OCHO$

dans laquelle R est un groupe formyle, hydroxyméthyle, formoxyméthyle, acétoxyméthyle ou nitrile, et le nombre total d'atomes de carbone dans les deux substituants alkyle est en tout au plus égal à 8, comme produits odoriférants ou ingrédients odoriférants de compositions de parfum, pour parfumages cosmétiques ou techniques.